# EUROPEAN PATENT APPLICATION

(11) **EP 4 342 499 A1**
(43) Date of publication of application: **27.03.2024**
(21) Application number: 22803761.0
(22) Date of filing: 26.04.2022
(51) Int. Cl.: A61K 49/06, A61K 49/00, A61B 5/00, A61B 5/055, C07D 403/14, C07D 209/60, C07F 5/00, C09K 11/06

(54) **FLUORESCENCE-MAGNETIC RESONANCE BIMODAL CONTRAST AGENT, PREPARATION METHOD THEREFOR AND USE THEREOF**

(30) Priority: 17.05.2021 CN 202110533217
(71) Applicant: Hainan Poly Pharmaceutical Co, .Ltd., Haikou, Hainan 571127 (CN); Zhejiang Poly Pharmaceutical Co. Ltd., Hangzhou, Zhejiang 311199 (CN); Anhui Poly Pharmaceutical Co., Ltd, Anqing, Anhui 246004 (CN); Zhejiang Longcharm Bio-Tech Pharma. Co., Ltd., Hangzhou, Zhejiang, 310018 (CN)
(72) Inventor: HAN, Yuxin, Hangzhou, Zhejiang 311199 (CN); ZHU, Yifan, Hangzhou, Zhejiang 311199 (CN); FAN, Minhua, Hangzhou, Zhejiang 311199 (CN); ZHANG, Zhen, Hangzhou, Zhejiang 311199 (CN); ZHU, Xinglong, Hangzhou, Zhejiang 311199 (CN)
(74) Representative: IPAZ
(86) International application number: PCT/CN2022/089377
(87) International publication number: WO 2022/242437

(57) **Abstract**

A fluorescence-magnetic resonance dual-modality contrast agent, a preparation method thereof and the use thereof. The contrast agent has the following structure: X-L-Y, wherein: formula (I); R₁, R₂, R₃, R₄, R₅, R₆, R₇, R₈, R₉, R₁₀, R₁₁, and R₁₂ are each independently selected from H, halogen, OH, NH₂, COOH, CONH₂, NO₂, CN, and low alkyl groups, the low alkyl groups can be substituted with halogen, OH, NH₂, COOH, CONH₂, SO₃H, NO₂, and CN, wherein R₃ and R₄, taken together with the carbon atoms to which they are attached, may form a phenyl group or a heterocyclic group; R₇ and R₈, taken together with the carbon atoms to which they are attached, may form a phenyl group or heterocyclic group; L is a linking group; and Y is a metal chelate.

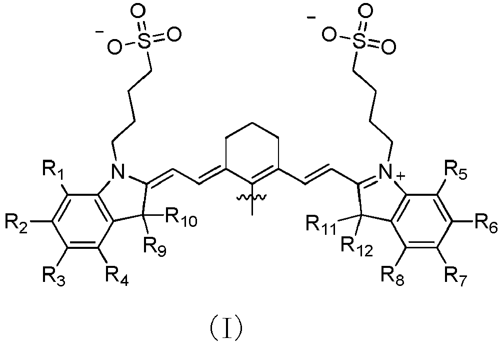

## Description

### Technical field

The present invention relates to the field of medical diagnostic imaging, more specifically, relates to a fluorescence-magnetic resonance dual-modality contrast agent, a preparation method, and a use thereof.

### Background

Recently, there has been significant progress in early disease diagnosis, especially for cancer. However, traditional clinical imaging technology is becoming insufficient for individualized cancer diagnosis and treatment. Traditional clinical diagnostic technologies, such as positron emission tomography (PET), computed tomography (CT), X-ray imaging, ultrasonic imaging (US), and magnetic resonance imaging (MRI), are being constrained by inherent shortcomings such as poor specificity and limited lesion location information. Wherein, MRI is the most widely used diagnostic imaging method in clinical practice. MRI technology usually utilizes the differences in the "spin-lattice relaxation time" and "spin-spin relaxation time" of protons in different tissues or organs for imaging. MRI is a non-invasive imaging method that provides anatomical, physiological, and molecular information about living animals without the use of ionizing radiation. The penetration depth of MRI is sufficient to image the entire human body, and the spatial resolution can reach below 10 µm according to the magnetic field intensity, MRI provides image information that is difficult to provide by other imaging means. While it is typically feasible to conduct MRI scans of the body's anatomical structure without the assistance of a contrast agent, obtaining a clear and precise image of tiny tumor lesions at the molecular level can be challenging due to the low sensitivity of MRI technology. This can impede the early detection and treatment of tumors, and as a result, a contrast agent may be required to enhance the quality of the tumor image. The limited sensitivity of commonly used MRI is associated with the signal detection mechanism and is difficult to overcome by its own improvement. The current commonly used MRI contrast agent gadopentetate dimeglumine (Gd-DTPA) has a low relaxation rate, and small molecule contrast agents are cleared quickly in the body. Therefore, improving the relaxation efficiency of MRI contrast agents is a key step in enhancing MRI contrast.

Optical imaging techniques utilize different physical parameters of light to interact with tissues for imaging, and many different optical imaging methods have been reported. These techniques rely on fluorescence, absorbed light, reflected light or bioluminescence as a source of contrast. Optical imaging technologies mainly include near-infrared fluorescence imaging (NIRF), reflectance imaging, and bioluminescence imaging. Despite its relatively recent appearance, various clinical and basic studies on optical imaging have progressed rapidly. The use of optical imaging technology offers several advantages. It is user-friendly, provides visual images, can perform multiple markings simultaneously, and is suitable for various spatial scales ranging from subcellular to tissue level. Moreover, it can be utilized for biological experiments, fluorescence-guided surgery, and endoscopic imaging. At the same time, most optical contrast agents are nontoxic, relatively cheap, universal and sensitive. Optical imaging can utilize a variety of organic or inorganic fluorescent contrast agents that fluoresce at different excitation wavelengths. The optical imaging for cancer diagnosis in the early stage is based on the change of endogenous fluorescence in tumor tissue. However, since it is difficult to distinguish the diagnostic signal components from the background fluorescence, exogenous contrast agents have to be developed to enhance the contrast of tumor tissue and normal tissue for fluorescence imaging. The main problem faced by optical imaging is difficult to reliably distinguish signals from background noise sounds of normal tissues, which may seriously affect the image quality. Meanwhile, poor tissue penetration depth and low spatial resolution are problems that optical imaging technology needs to solve. Indocyanine green (ICG) is currently the first-line clinical fluorescent contrast agent. Initially, it is used as a dye drug to assess liver function and effective blood flow to the liver, but now widely used for intraoperative navigation in liver surgery and lymph node clearance in breast cancer. After the ICG is injected intravenously into the body, it is immediately combined with plasma protein, rapidly distributed in blood vessels of the whole body along with the blood circulation. It is efficiently and selectively taken up by hepatocytes, and then released in a free form from the liver cells into the bile, and then enters into the intestine through the bile duct, and is discharged out of the body with the feces. Due to its rapid excretion, about 97% of it is eliminated from the blood after 20 minutes of intravenous injection in normal people, it does not take part in chemical reactions in the body, there is no enterohepatic circulation, there is no lymphatic reflux and it is not excreted by other extrahepatic organs such as the kidneys. Due to the structure of the ICG, sterile water for injection is the preferred solvent, but the stability of the aqueous solution is poor. In addition, in the process of guiding the operation, due to the conditions of liver cirrhosis and other conditions, the indocyanine green can present false positives in the liver, reducing the accuracy of liver resection surgery.

Therefore, providing more comprehensive diagnostic information is beneficial for improving diagnostic accuracy. Purely morphological images alone may not provide sufficient information. On the other hand, optical imaging technology, PET, single photoelectron emission computed tomography (SPECT), and other high-sensitivity technologies that can provide more information outside the image are limited by the defect of low spatial resolution. Although the simultaneous use of multiple contrast agents alone can result in superior contrast results in each imaging modality, the establishment of the bimodal contrast agent can help ensure that the pharmacokinetics of signals of each mode are the same, co-positioning can be carried out, and can also avoid additional stress on the body's blood clearance mechanisms. When designing multimodal contrast agents, attention should be paid to avoid overlapping the functions and advantages of the selected imaging modes, and to compensate for the weaknesses of each imaging modality to maximize synergy.

### Summary

With regard to the deficiency existing in the prior art, the present invention provides a fluorescence-magnetic resonance dual-modality contrast agent, wherein the contrast agent has the following structure:

X-L-Y,

wherein:
X has the following structure: 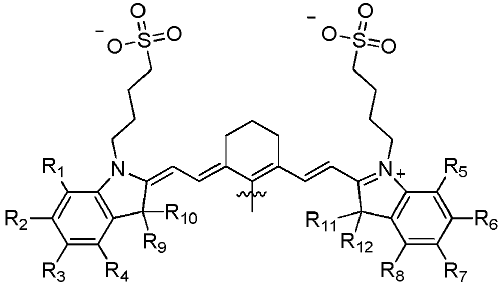
R₁, R₂, R₃, R₄, R₅, R₆, R₇, R₈, R₉, R₁₀, R₁₁, and R₁₂ are each independently selected from H, halogen, OH, NH₂, COOH, CONH₂, NO₂, CN, and low alkyl groups, wherein the low alkyl groups can be substituted with halogen, OH, NH₂, COOH, CONH₂, SO₃H, NO₂, or CN, wherein:
   R₃ and R₄, taken together with the carbon atoms to which they are attached, may form a phenyl group or a heterocyclic group; and R₇ and R₈, taken together with the carbon atoms to which they are attached, may form a phenyl group or a heterocyclic group;
   wherein the halogen is selected from fluorine, chlorine, bromine, and iodide ion; and
   wherein the low alkyl is selected from the C₁₋₆ alkyl, such as methyl, ethyl, n-propyl, isopropyl, tert-butyl, n-butyl, pentyl, cyclopropyl, cyclopentyl, and cyclohexyl.

Preferably, the said low alkyl is selected from the C₁₋₃ alkyl, such as methyl, ethyl, n-propyl, and isopropyl.

The dual-modality contrast agent has a good ability of magnetic resonance T1-weighted imaging contrast and living body fluorescence imaging and has a high relaxation rate.

Preferably, the X has the following structure: 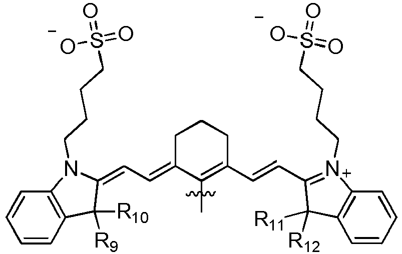 wherein, R₉, R₁₀, R₁₁ and R₁₂ are as defined above.

Further preferably, the X has the following structure:

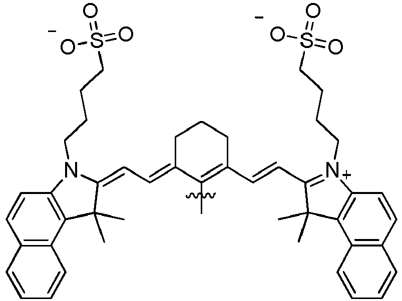

L is a linking group, having a structure according to the following formula: 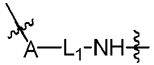
wherein A is selected from the group consisting of S, N, and O, preferably, A is N or O;
L₁ is a carbon chain containing 8 to 20 carbon atoms, wherein the carbon atom(s) in the carbon chain may be replaced with oxygen atom(s) or nitrogen atom(s); the carbon chain can contain double bond(s); the hydrogen atom(s) in the carbon chain can be substituted with 1-5 R₁₃, wherein the each R₁₃ is independently selected from benzyl, carboxyl, and C₁₋₃ alkyl, or two adjacent R₁₃ can form a ring, taken together with the atoms to which they are attached, exemplary such as forming a ring: 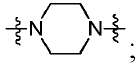 and the carbon(s) in the carbon chain can be further replaced with carbonyl group(s), for example, the carbon chain comprises the group:

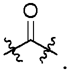

Further preferably, L has the following structure: 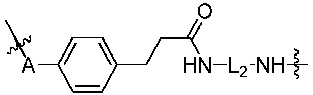
wherein, A is as defined above.
L₂ has the same definition as L₁.

The dual-modality contrast agent has the advantages of a high relaxation rate, excellent thermal stability, low toxicity, and good fluorescence and magnetic resonance signal correspondence, which can be effectively used for magnetic resonance imaging and fluorescence imaging of living cells and living bodies.

Further preferably, L₂ has the following formula: 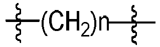 wherein n is 8-20, more preferably n is 10-15.

The fluorescence-magnetic resonance dual-modality contrast agent provided by the invention uses magnetic resonance signals to monitor the distribution and metabolism of fluorescent dyes in human tissues in real time, and it has been verified by a large number of experiments that some of the linking groups are easy to be decomposed, which can cause the two components to be separated very quickly in the body, affecting the final monitoring effect. When the L₂ is selected from the following formula: 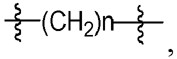 the obtained fluorescence-magnetic resonance dual-modality contrast agent is stable and can achieve excellent detection results, which is expected to meet clinical needs.

Y is a metal chelate.

Preferably, the metal chelates complexes with Gd to form a Gd complex (Z).

Further preferably, the Gd complex has the following structures:

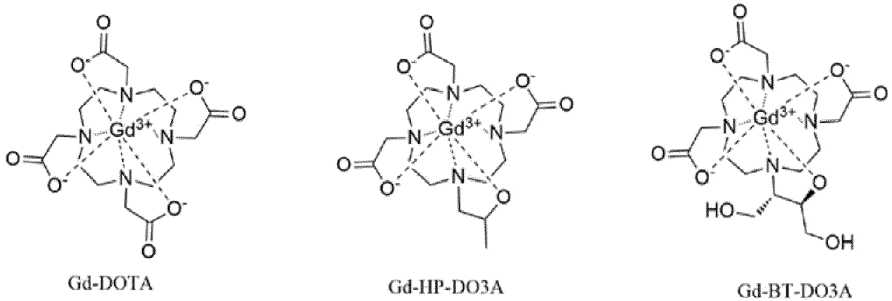

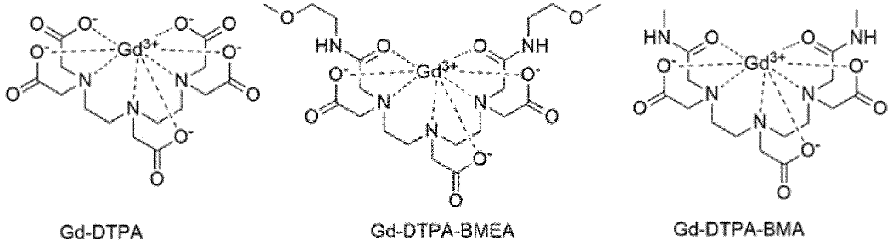

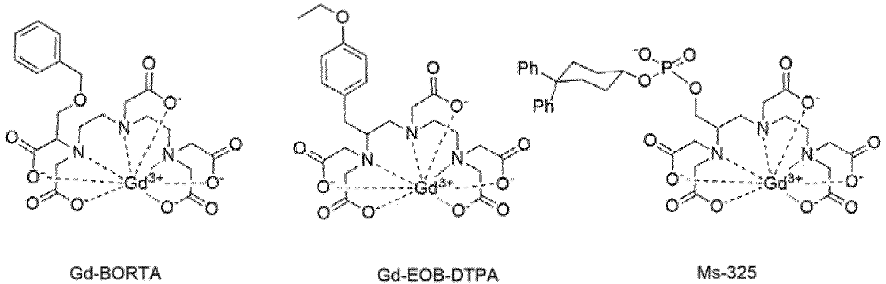

Further preferably, the fluorescence-magnetic resonance dual-modality contrast agent of the present invention has the following structures:

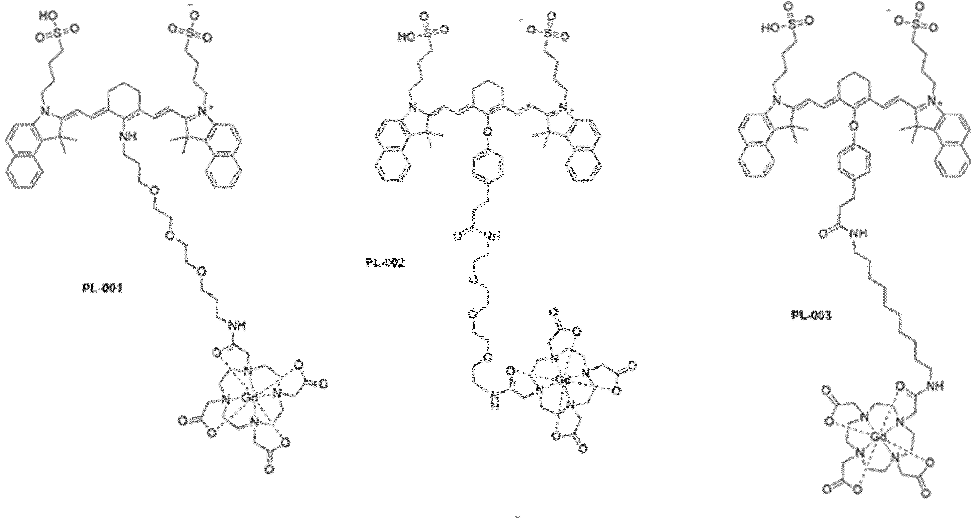

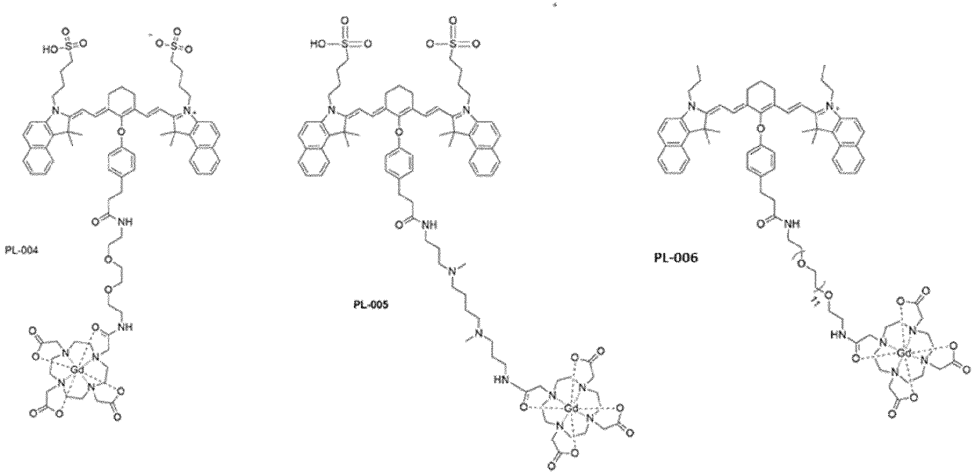

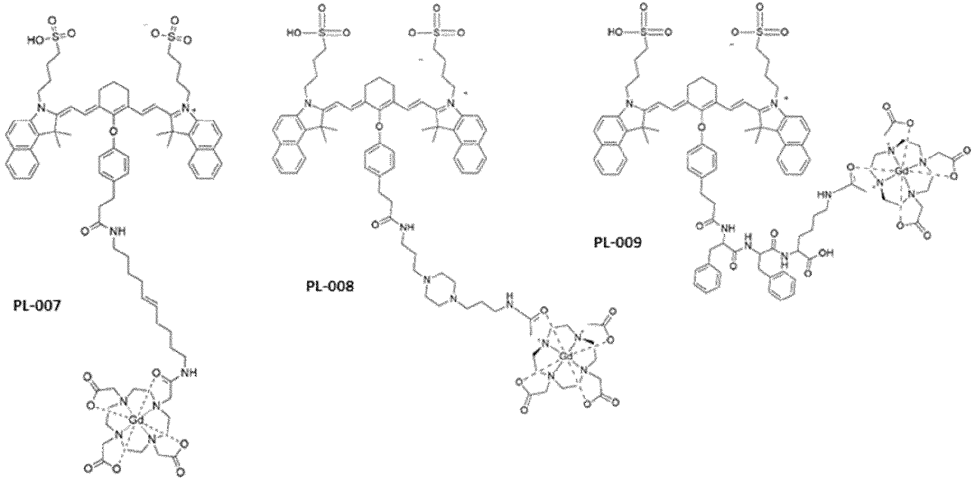

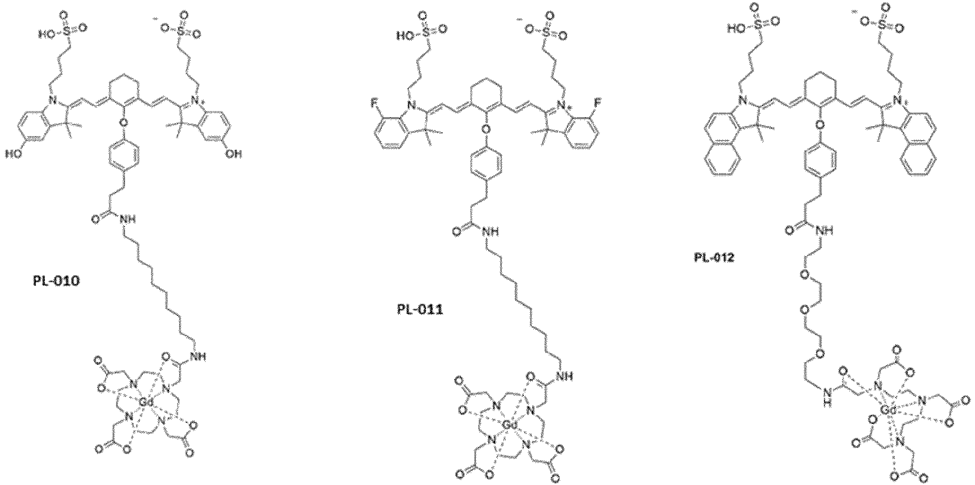

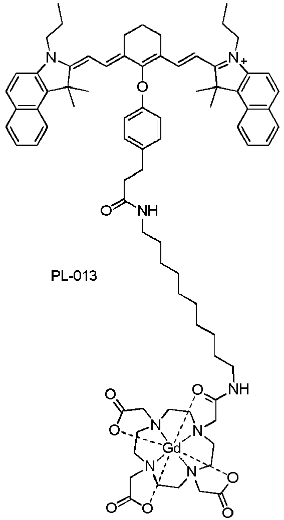

In another aspect, the invention provides a method for preparing the fluorescence-magnetic resonance dual-modality contrast agent, and the reaction equation is as follows:

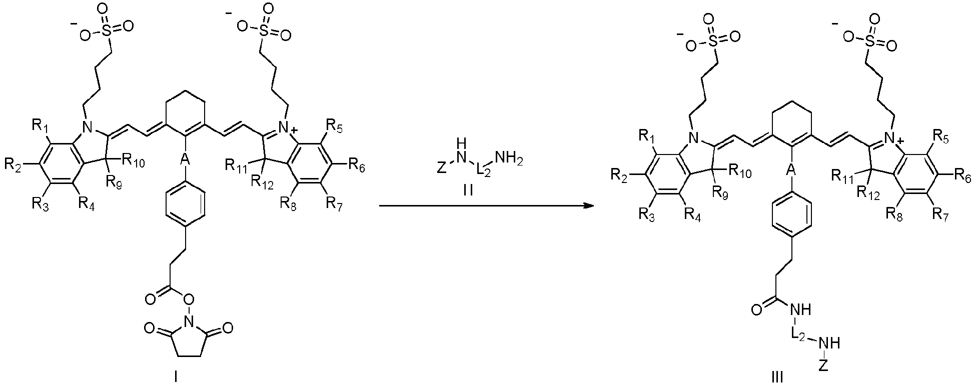

The method for the preparation of a fluorescence-magnetic resonance dual-modality contrast agent of formula III comprises the step of condensing a compound of formula I with a compound of formula II;
wherein the compound of formula II is obtained by complexing a compound of formula 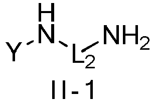 with gadolinium chloride hydrate; and
wherein the definitions of R₁, R₂, R₃, R₄, R₅, R₆, R₇, R₈, R₉, R₁₀, R₁₁, R₁₂, Y, A, L, and Z are the same as above.

Alternatively, the fluorescence-magnetic resonance dual-modality contrast agent of formula III-2 provided by the present invention can also be obtained by complexing a compound of formula III-1 with gadolinium chloride hydrate, and the reaction equation is as follows: 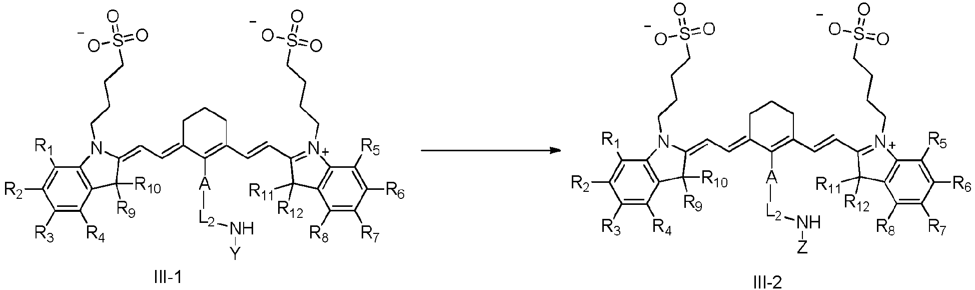
wherein a compound of formula 1-2 is condensed with a compound of formula II-2 to obtain the compound of formula III-1, and its reaction equation is as follows: 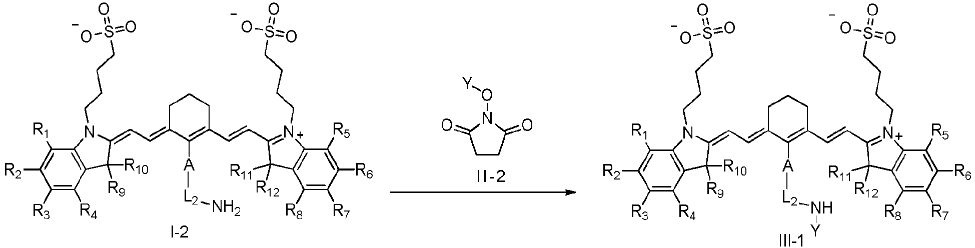
wherein, the definitions of R₁, R₂, R₃, R₄, R₅, R₆, R₇, R₈, R₉, R₁₀, R₁₁, R₁₂, A, L₂, and Y are the same as above.

At the same time, the present invention provides the use of the fluorescence-magnetic resonance dual-modality contrast agent, the use is mainly for medical diagnosis, specifically for medical magnetic resonance enhanced imaging, determination of liver function, assisting in preoperative planning, intraoperative fluorescence navigation, prediction of fluorescence distribution of viscera by magnetic resonance images, determination of liver function, determination of renal function, monitoring of the vivo circulation as well as labeling of cells, and markers for in vitro cellular analysis.

The beneficial effects of the present invention include the following: the dual-modality contrast agent provided by the present invention has good capability of magnetic resonance T1-weighted imaging and living body fluorescence imaging, high relaxation rate, excellent thermal stability, low toxicity, good fluorescence and magnetic resonance signal correspondence, which can be effectively used for magnetic resonance imaging and fluorescence imaging of living cells and living bodies. And can still significantly enhance the magnetic resonance signals of the liver and kidney at 6 hours post-dosing, significantly improving the resolution of organ boundaries. The intensity of magnetic resonance signals has a high correspondence with the fluorescence intensity, and it has the desired effect of tumor diagnosis and surgical navigation. It has great potential in clinical application.

### Brief description of the drawings

Figure 1(A) is the compound PL-001 in mice magnetic resonance T1 enhancement imaging;
Figure 1(B) is the bar chart of the mean signal values of T1-enhanced liver and kidney magnetic resonance images;
Figure 1(C) is the liver and kidney fluorescence imaging images after 6h of PL-001 administration;
Figure 1(D) is the liver and kidney fluorescence efficiency values bar chart.
Figure 2 is the results of flow cytometry to measure the rate of contrast agent entry in H22 cells.
Figure 3 is cross-sectional images of magnetic resonance T1-enhanced imaging of mice liver in situ tumors before and after contrast agent injection, with tumor tissue in the dashed line.
Figure 4 is the results of fluorescence imaging of major tissues in mice with liver in situ tumors.
Figure 5 is the PL-003 HPLC purity chromatogram.

### Detailed description of the embodiments

In order to better understand the technical solution of the present invention, the present invention is further illustrated by following embodiments. The embodiments are intended to facilitate understanding of the present invention and should not be deemed as a limitation to the present invention.

### Embodiment 1: Preparation of compound PL-001

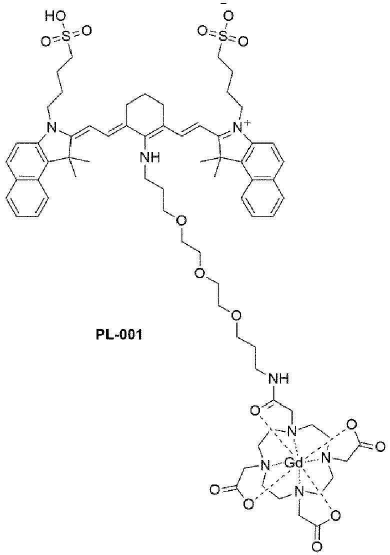

The synthesis scheme is as follows:

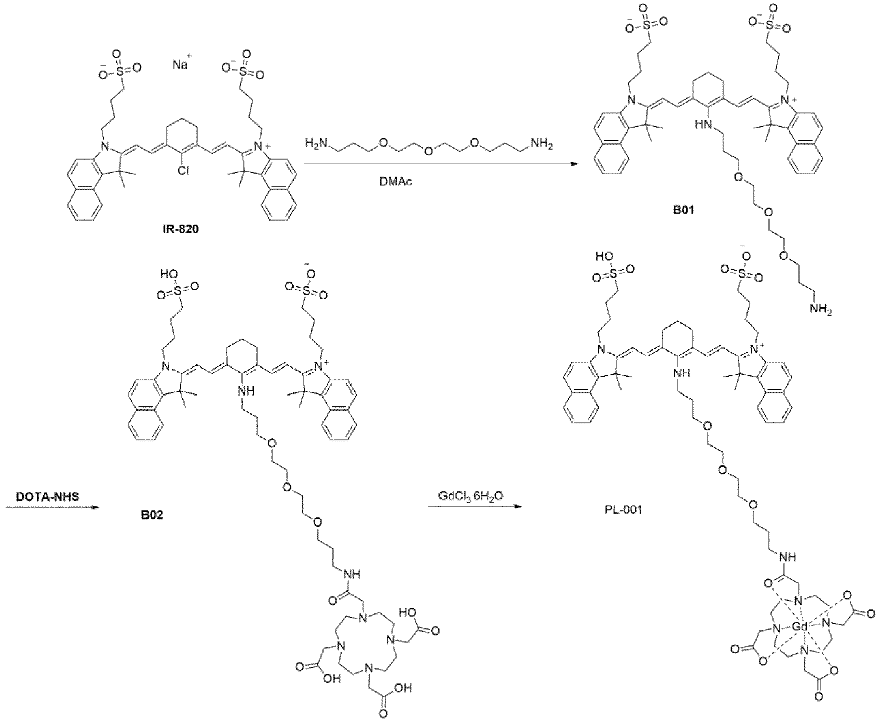

### Reaction steps:

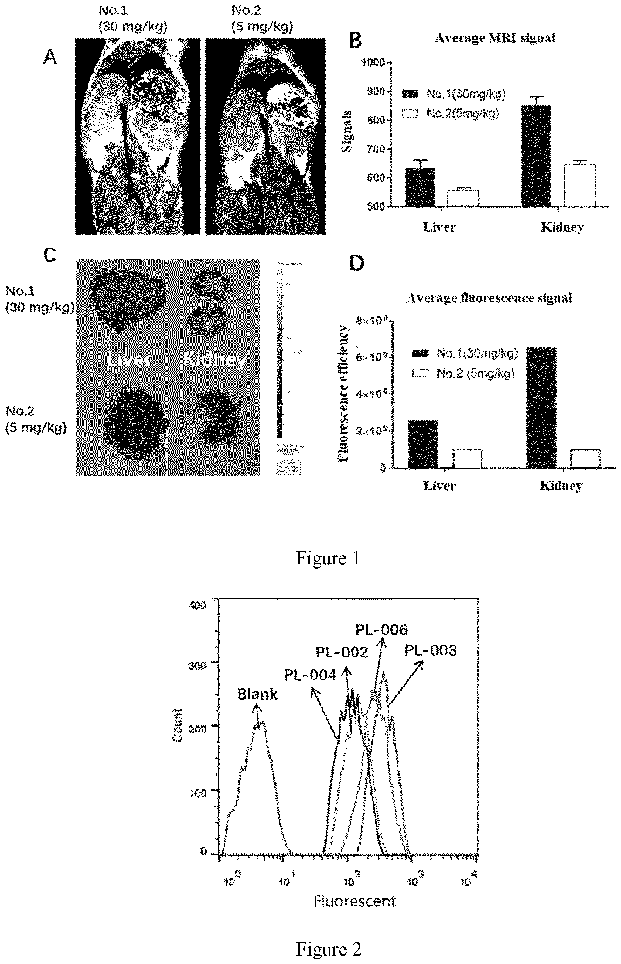

1. IR-820 (new indocyanine green) and 3,6,9-trioxaundecamethylenediamine (1:1-1:5 eq.) were added to the DMSO solution. And then the triethylamine as the deacid reagent was added to the mixture. The mixture reacted at 20-80°C for 2h. The desired product B01 was obtained after precipitation.
2. B01 and DOTA-NHS (NBS is N-hydroxysuccinimide) (1:1-1:3 eq.) were dissolved in DMSO solution, and 1-20 equivalents of triethylamine was added to the mixture. The mixture reacted at 10-40°C for 24h. The desired product B02 was obtained after precipitation.
3. B02 was reacted with 0.5-2 equivalents of gadolinium chloride hydrate in DMSO at 10-50°C for 10h-50h, and the desired product PL-001 was obtained after precipitation, yield of 83%, purity of 91%.
4. The final product was dissolved in pure water to create a solution. The product was administered to ICR mice by tail intravenous injection at doses of 5 mg/kg and 30 mg/kg, respectively. Magnetic resonance T1-enhanced imaging (SE sequence) was performed on the liver and kidney of the mice before and after the administration of the product. After the magnetic resonance imaging was completed, the livers and kidneys were removed for fluorescence imaging. The obtained images were analyzed by ImageJ software, and the results are shown in Figure 1: A is the compound PL-001 in mice magnetic resonance T 1-enhanced imaging, with significant enhancement of liver and kidney; Figure B is the bar chart of the mean signal value of the T1-enhanced liver and kidney magnetic resonance image; Figure C is the fluorescence imaging image of the liver and kidney 6 hours after administration of PL-001, with significant fluorescence of the liver and kidney; and Figure D is the value of liver and kidney fluorescence efficiency bar chart. From the figure, it can be seen that the magnetic resonance enhancement and fluorescence imaging results of the liver and kidney were basically consistent.

### Embodiment 2: Preparation of compound PL-002

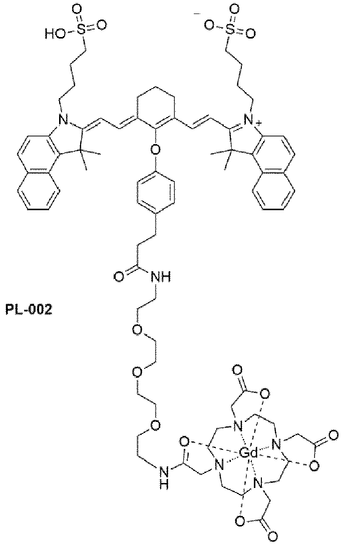

The synthesis scheme is as follows:

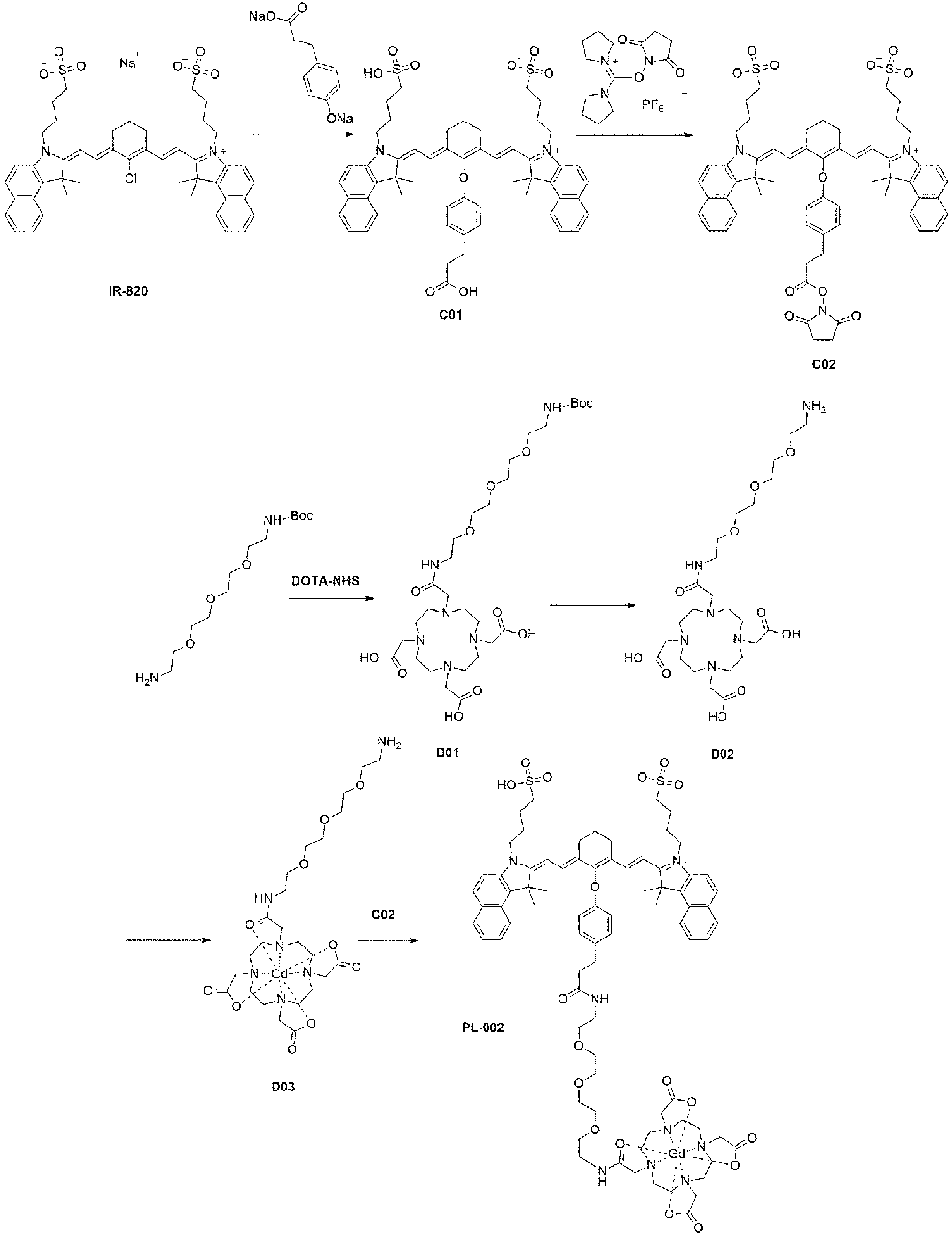

### Reaction steps:

1. IR-820 and sodium p-oxide phenylpropionate (1:1-1:5 eq.) were dissolved in DMSO and reacted at 20-80°C for 1-8h. The desired intermediate C01 was obtained after precipitation.
2. C01 and dipyrrolidino(N-succinimidyloxy)carbenium hexafluorophosphate (1:05-1:3 eq.) were dissolved in DMSO, 1 equivalent of N, N-diisopropylethylamine was added, and the mixture reacted at 10-50°C for 10-30 h. The desired intermediate C02 was obtained after precipitation.
3. 5,8,11-Trioxa-2-azatridecanoic,13-amino,1,1-dimethylethyl ester and DOTA-NHS (1:1-1:3 eq.) were dissolved in DMSO, 1-3 equivalents of triethylamine was added to the mixture. The reaction reacted at room temperature for 24h. The desired intermediate D01 was obtained after precipitation.
4. D01 was added to a mixed trifluoroacetic acid and dichloromethane solution and reacted at 0-40°C for 24h. The desired intermediate D02 was obtained after ether precipitation.
5. D02 and gadolinium chloride hydrate (1:0.5-1:3 eq.) were dissolved in water and reacted at room temperature for 1-5 days, and the intermediate D03 was obtained after purification by column chromatography.
6. D03 and C02 (1:0.5-1:3eq.) were dissolved in DMSO, 1-5 equivalents of N, N-diisopropylethylamine was added to the mixture. The reaction reacted at 0-50°C for 8h and the desired product PL-002 was obtained after purification by column chromatography, purity of 91.2%, yield of 55%.

### Embodiment 3: Preparation of compound PL-003

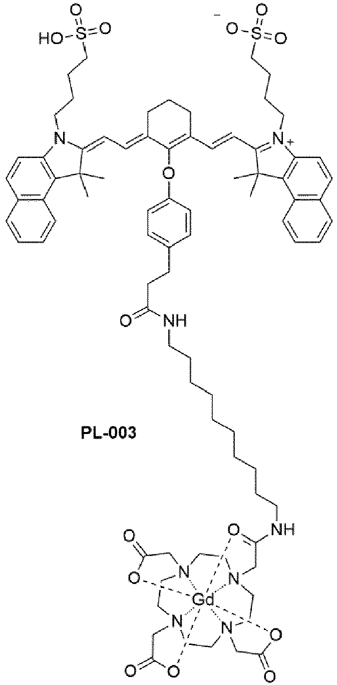

The synthesis scheme is as follows:

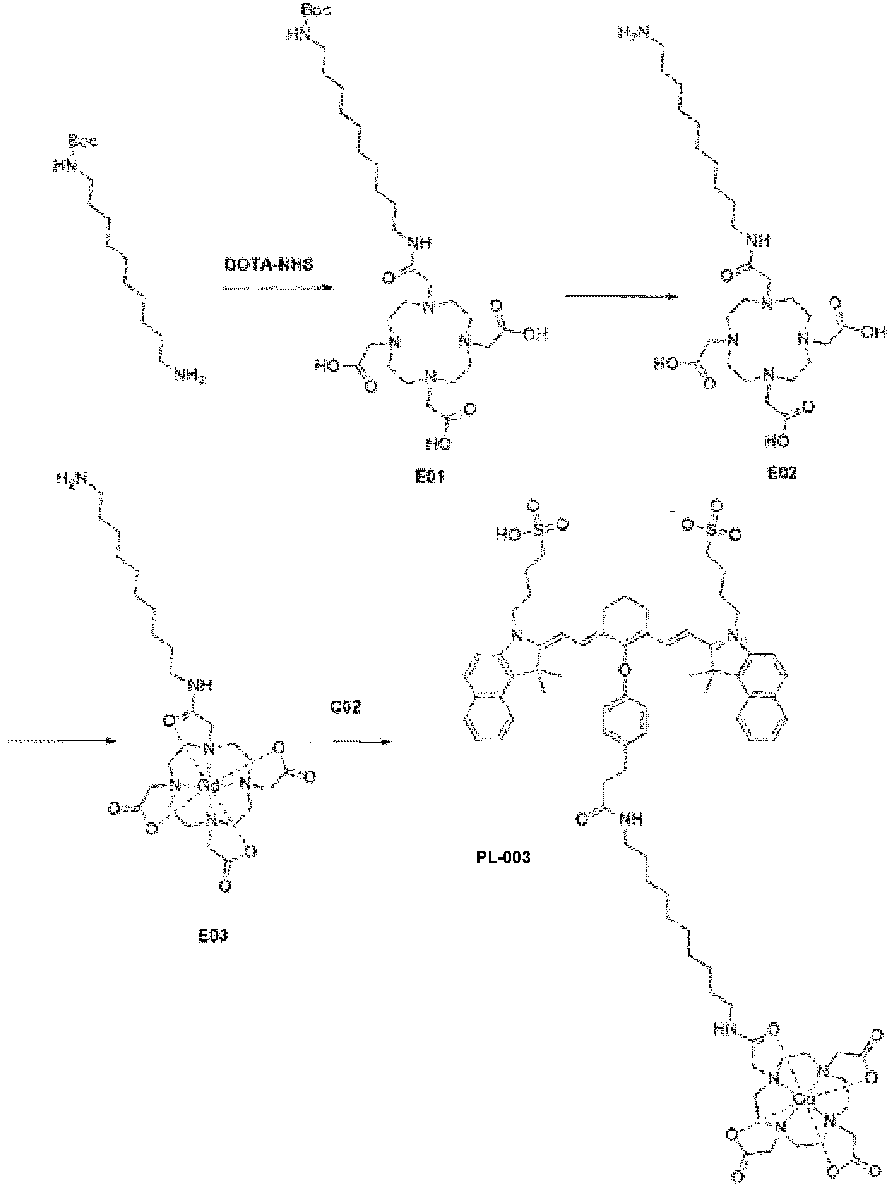

### Reaction steps:

N-Boc-1,10-diaminodecane and DOTA-NHS (1:1-1:3eq.) were dissolved in DMSO, and 1-3 equivalents of triethylamine was added and reacted at room temperature for 24h. The desired intermediate E01 was obtained after precipitation.

D01 was added to a mixed solution of trifluoroacetic acid and dichloromethane and reacted at 0-40°C for 24h, and the desired intermediate E02 was obtained after ether precipitation.

D02 and gadolinium chloride hydrate (1:0.5-1:3 eq.) were dissolved in water and reacted at room temperature for 1-5 days, and the intermediate E03 was obtained after purification by column chromatography.

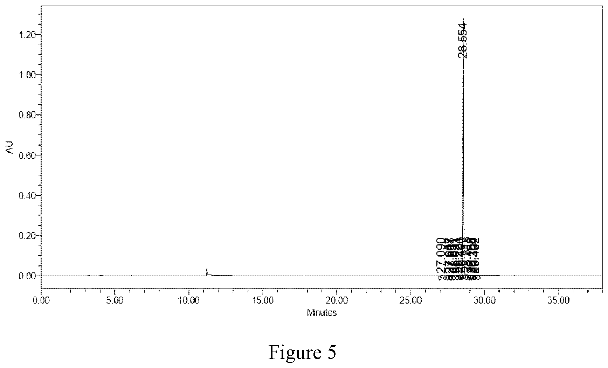

D03 and C02 (1:0.5-1:3 eq.) were dissolved in DMSO, and 1-5 equivalents of N, N-diisopropylethylamine was added, and the reaction reacted at 0-50°C for 8 h. The desired product PL-003 was obtained after purification by column chromatography, purity of 96.3%, yield of 51%, and its HPLC chromatogram is shown in Fig. 5, and its corresponding data are shown in Table 1:

**Table 1**

| number | retention time | peak area | %peak area |
|---|---|---|---|
| 1 | 27.09 | 8450 | 0.24 |
| 2 | 27.647 | 2744 | 0.08 |
| 3 | 27.698 | 2510 | 0.07 |
| 4 | 27.851 | 8510 | 0.24 |
| 5 | 28.021 | 13452 | 0.38 |
| 6 | 28.224 | 14853 | 0.42 |
| 7 | 28.4 | 20736 | 0.58 |
| 8 | 28.554 | 3438604 | 96.3 |
| 9 | 28.815 | 32122 | 0.9 |
| 10 | 29.108 | 6220 | 0.17 |
| 11 | 29.166 | 9161 | 0.26 |
| 12 | 29.304 | 3534 | 0.1 |
| 13 | 29.402 | 9883 | 0.28 |

The HPLC method was as follows: flow rate of 0.8 mL/min, injection volume of 10 µL, detection wavelength of 254 nm, a chromatographic column of Xtimate C18 Welch with 250×4.6 mmx3 um, eluent A was a mixed solvent (pH 6.0) of 120 mM acetamide and 5 mM citric acid solution, eluent B was acetonitrile, and the diluent was methanol, the flow phase gradient was as follows:

| times/min | A% | B% |
|---|---|---|
| 0 | 100 | 0 |
| 4 | 100 | 0 |
| 8 | 80 | 20 |
| 13 | 80 | 20 |
| 16 | 70 | 30 |
| 21 | 70 | 30 |
| 26 | 30 | 70 |
| 29 | 30 | 70 |
| 33 | 100 | 0 |
| 38 | 100 | 0 |

### Embodiment 4: Preparation of compound PL-004

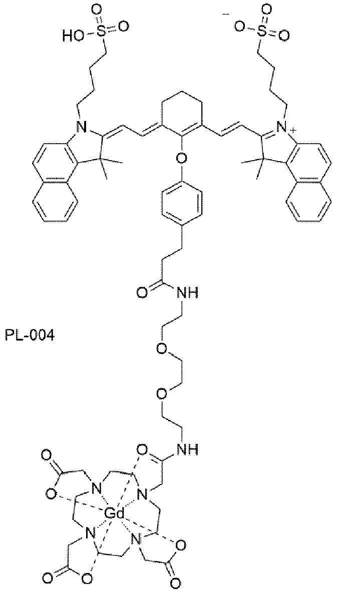

The synthesis scheme is as follows:

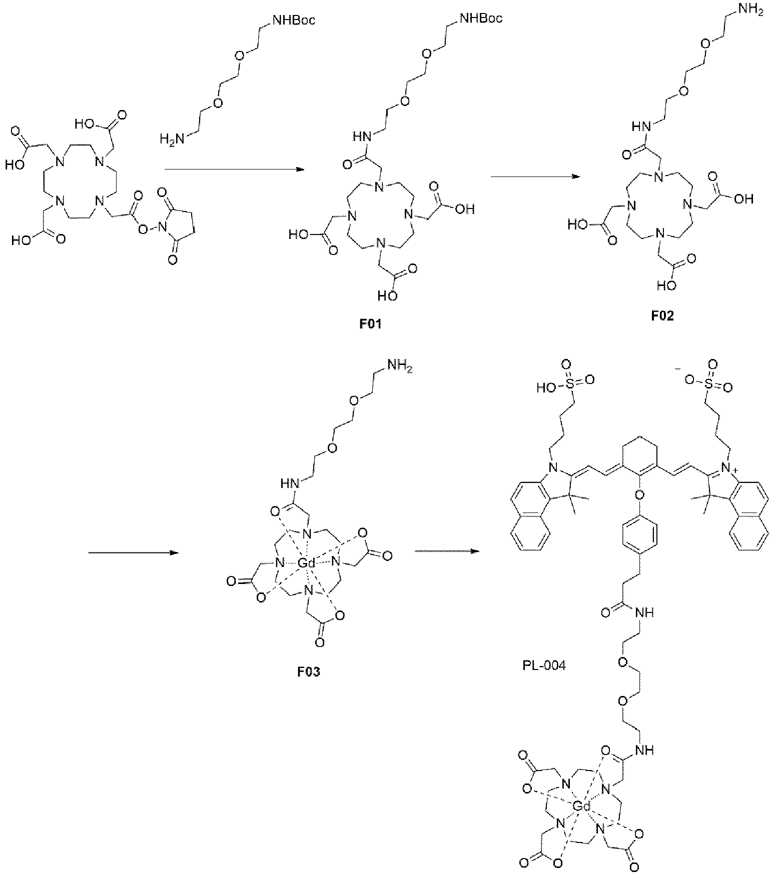

### Reaction steps:

N-Boc-2,2'-(ethylenedioxy)bis(ethylamine) and DOTA-NH (1:1-1:3eq.) were dissolved in DMSO, 1-3 equivalents of triethylamine was added and reacted at room temperature for 24h. The desired intermediate F01 was obtained after precipitation;
F01 was added to the mixed solution of trifluoroacetic acid and dichloromethane, reacted at 0-40°C for 24h, and the desired intermediate F02 was obtained after ether precipitation;
F02 and gadolinium chloride hydrate (1:0.5-1:3eq.) were dissolved in water and reacted at room temperature for 1-5 days, and the intermediate F03 was obtained after purification by column chromatography.
F03 and C02 (1:0.5-1:3 eq.) were dissolved in DMSO, 1-5 equivalents of N, N-diisopropylethylamine was added, reacted at 0-50°C for 8h, and the desired product PL-003 was obtained, purity of 93.3%, yield of 43%.

### Embodiment 5: Preparation of compound PL-005

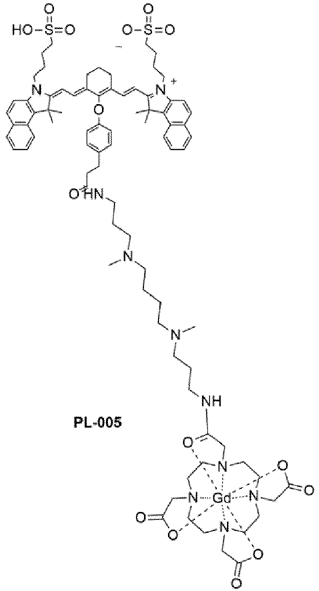

The synthesis scheme is as follows:

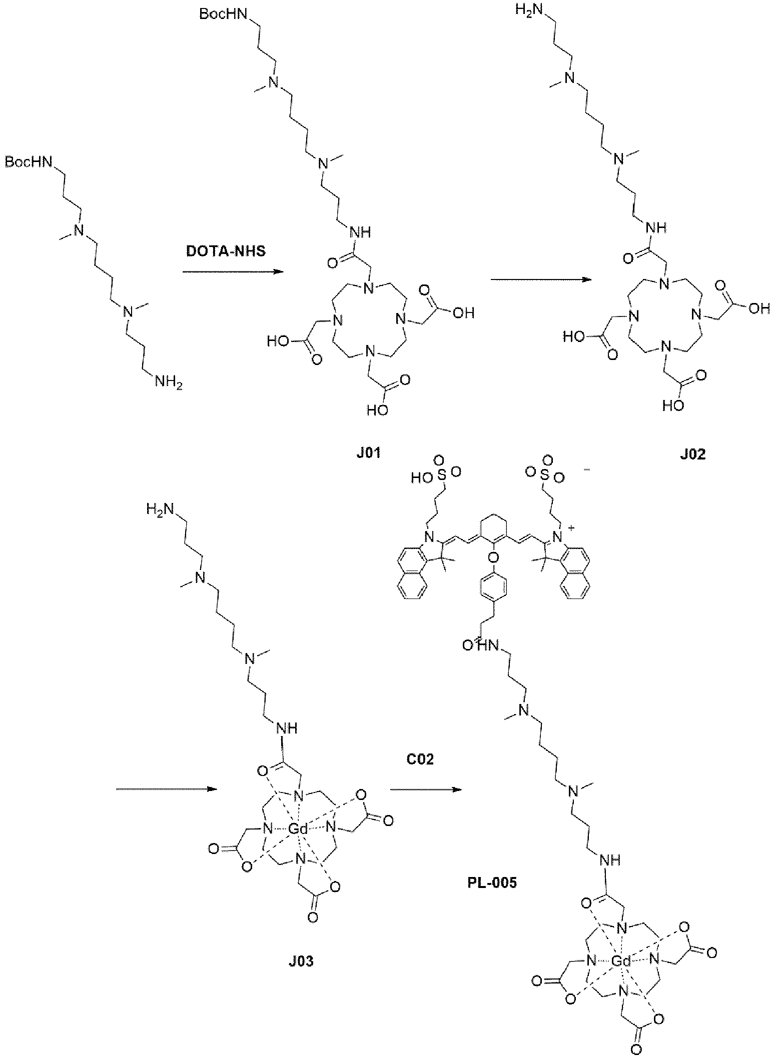

### Reaction steps:

N1-Boc-N4-N9-Dimethylspermine and DOTA-NHS (1:1-1:3eq.) were dissolved in DMSO,1-3 equivalents of triethylamine was added, reacted at room temperature for 24h, and the desired intermediate J01 was obtained after precipitation;
J01 was added to the mixed solution of trifluoroacetic acid and dichloromethane, reacted at 0-40°C for 24h, and the desired intermediate J02 was obtained after ether precipitation.
J02 and gadolinium chloride hydrate (1:0.5-1:3 eq.) were dissolved in water and reacted at room temperature for 1-5 days, and the intermediate J03 was obtained after purification by column chromatography;
J03 and C02 (1:0.5-1:3 eq.) were dissolved in DMSO, 1-5 equivalents of N, N-diisopropylethylamine was added, reacted at 0-50°C for 8h, and the desired product PL-005 was obtained after purification by column chromatography;

The compounds obtained in Examples 6-13 in Table 2 below were prepared according to the method of Embodiment 2 after replacing the reaction intermediates.

**Table 2**

| Embodiments | Compounds | Purity | Yield |
|---|---|---|---|
| 6 | 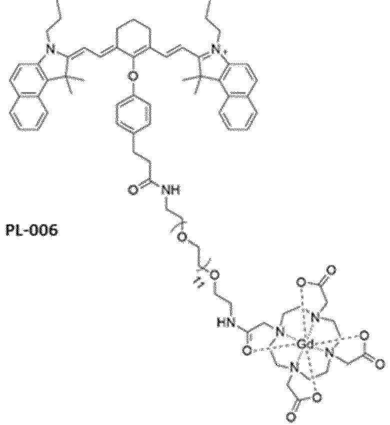 | 92.1% | 43.1% |
| 7 | 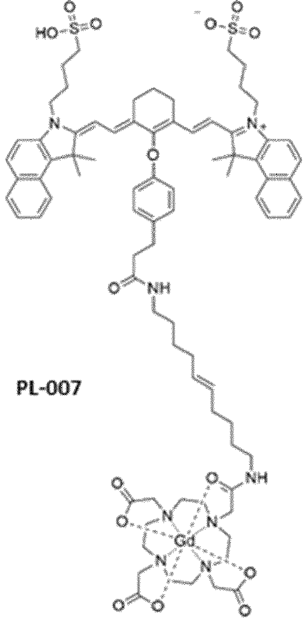 | 88.5% | 55.3% |
| 8 | 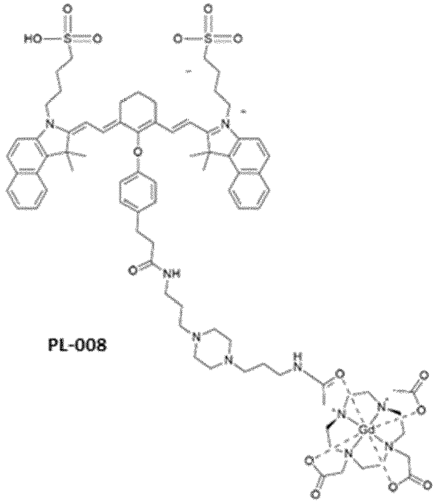 | 92.2% | 60.9% |
| 9 | 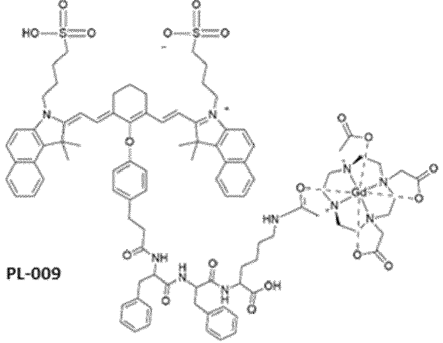 | 82.1% | 45.3% |
| 10 | 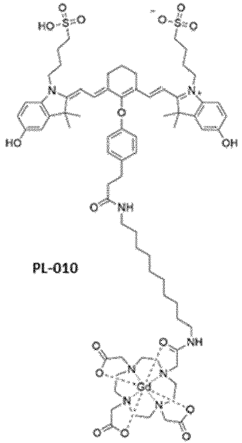 | 86.7% | 65.2% |
| 11 | 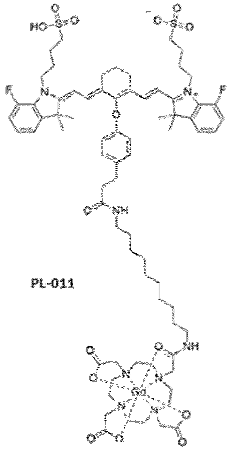 | 92.8% | 43.7% |
| 12 | 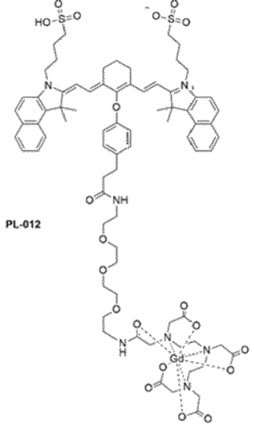 | 88.3% | 49.8% |

After preparation of PL-005, PL-008, and PL-013, it was found that this compound was easily soluble in highly polar organic solvents (methanol, N, N-dimethylformamide, dimethyl sulfoxide, etc.), and difficult to soluble in water (<0.1 mg/mL).

### Embodiment 13:

The longitudinal relaxation rate (r1) of the contrast agent was determined in a 0.35T small nuclear magnetic resonance imaging system using an IR reaction sequence. The relaxation rates of the contrast agent in pure water and in bovine serum protein solution (1%, w/w) in different solvents are shown in Table 3 below.

**Table 3**

| | **r₁ in Water (mM^{- 1}s⁻¹)** | **r₁ in BSA solution(1%) (mM⁻¹s⁻¹)** |
|---|---|---|
| **PL-002** | 11.8 | 16.2 |
| **PL-003** | 15.4 | 18.9 |
| **PL-004** | 14.7 | 17.6 |
| **PL-006** | 14.9 | 17.5 |
| **PL-007** | 15.2 | 18.6 |
| **PL-009** | 15.5 | 19.1 |
| **PL-010** | 14.7 | 17.9 |
| **PL-011** | 14.3 | 18.5 |
| **PL-012** | 10.9 | 14.6 |

### Embodiment 14:

The aqueous solution of the contrast agent (0.1 mg/mL) was left at room temperature and its stability was measured by calibrating its relative content by the HPLC method at different time points, and the stability of the contrast agent is shown in Table 4.

**Table 4**

| | **PL-001** | **PL-002** | **PL-003** | **PL-004** | **PL-006** |
|---|---|---|---|---|---|
| **decomposition rate%** | | | | | |
| **0h** | 0 | 0 | 0 | 0 | 0 |
| **2h** | 5.9 | <LOQ | <LOQ | <LOQ | <LOQ |
| **24h** | 40.0 | <LOQ | <LOQ | <LOQ | <LOQ |
| **48h** | 65.4 | <LOQ | <LOQ | <LOQ | <LOQ |
| | | | | | |

| | **PL-007** | **PL-009** | **PL-0010** | **PL-0011** | **PL-0012** |
|---|---|---|---|---|---|
| **decomposition rate%** | | | | | |
| **0h** | 0 | 0 | 0 | 0 | 0 |
| **2h** | <LOQ | <LOQ | <LOQ | <LOQ | <LOQ |
| **24h** | <LOQ | <LOQ | 1.5 | 4.7 | <LOQ |
| **48h** | <LOQ | <LOQ | 5.4 | 10.5 | <LOQ |

### Embodiment 15:

The maximum tolerated dose (MTD) of the contrast agent was preliminarily determined by administration to mice by tail intravenous injection. Four ICR female mice (8 weeks old) per group were selected for tail intravenous injection at doses of 25, 50, 75, 100, 125, and 150 mg/kg and were continuously observed for a week, and the results are shown in Table 5.

**Table 5**

| | MTD mg/kg |
|---|---|
| ICG | 60* |
| PL-001 | 75 |
| PL-002 | 125 |
| PL-003 | 150 |
| PL-004 | 125 |
| PL-006 | 100 |
| PL-007 | 150 |
| PL-009 | 100 |
| PL-010 | 125 |
| PL-011 | 75 |
| PL-012 | 150 |

### Embodiment 16:

The cell entry rate of the contrast agent at the cell level was determined by flow cytometers. Logarithmically grown murine hepatocellular carcinoma cells H22 were collected and spread evenly in 12-well plates, with an approximate cell number of 1.5 × 105 per well. After wall attachment was complete for 24 h, PBS, PL-002, PL-003, PL-004, and PL-006 were added to each well, respectively, and the cells were trypsin-digested after a certain time of incubation and washed twice with PBS, and then finally resuspended in 0.5 mL of PBS solution, and their cell entry was detected by flow cytometry. The results are shown in Figure 2, PL-003 has the fastest cellular entry.

### Embodiment 17:

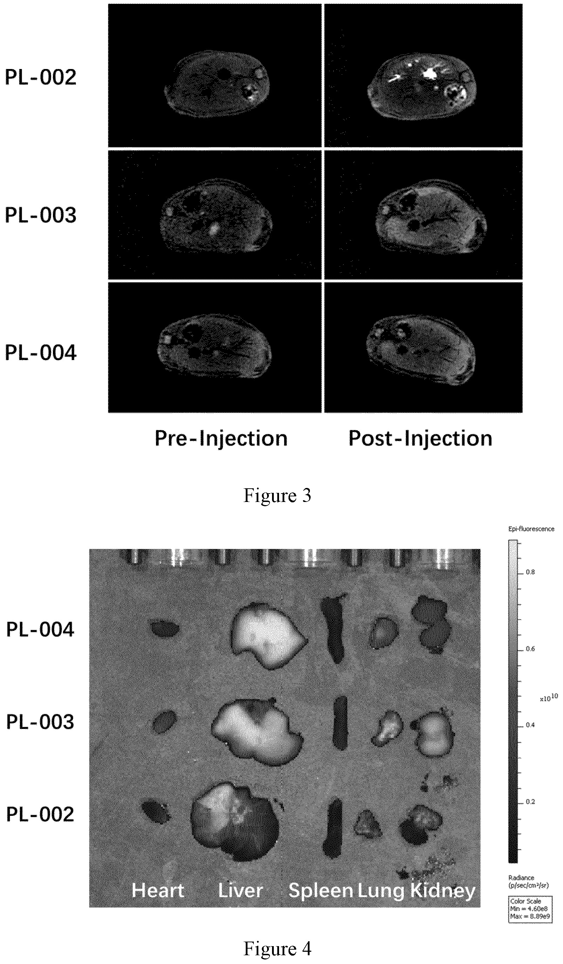

Magnetic resonance imaging of liver in situ tumors with a contrast agent was performed using a GE 3.0T magnetic resonance imaging system. H22 cells were inoculated in Ba1b/c mice through liver in-situ injection, and liver conditions were observed through magnetic resonance. After the tumor was formed, 1mg/mL contrast agent solution (5mL/kg) was injected through the tail intravenous, and the liver was subjected to magnetic resonance T1-enhanced imaging at different time points after the administration of the agent. The results are shown in Figure 3. The imaging effect of the tumor site can be significantly enhanced after the injection of the contrast agent, and PL-003 has a better enhancement effect.

### Embodiment 18:

Liver in situ tumor mice tissue fluorescence was observed by a small animal live imaging analyzer (Perkin Elmer). After magnetic resonance imaging, the major organs of the mice were removed and observed by live imaging, and the results are shown in Figure 4. All of the contrast agents have more obvious fluorescence in the liver, and the main fluorescence range coincides with the magnetic resonance imaging results, in which the PL-003 tumor is distinguished from the normal tissue and has better fluorescence intensity. This figure indicates that the contrast agent of the present invention can effectively improve the distinction between normal tissue and tumor tissue in imaging, which is helpful in assisting preoperative planning and intraoperative fluorescence navigation.

## Claims

1. A fluorescence-magnetic resonance dual-modality contrast agent, wherein the contrast agent has the following structure:
X-L-Y,
wherein:
X has the following structure: 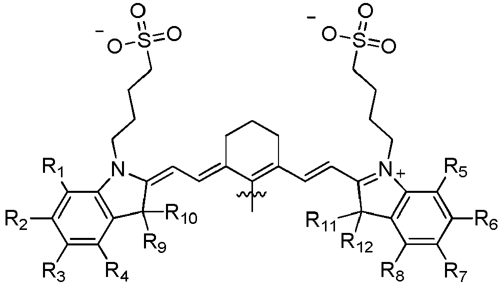
R₁, R₂, R₃, R₄, R₅, R₆, R₇, R₈, R₉, R₁₀, R₁₁, and R₁₂ are each independently selected from H, halogen, OH, NH₂, COOH, CONH₂, NO₂, CN, and low alkyl groups, wherein the low alkyl groups can be substituted with halogen, OH, NH₂, COOH, CONH₂, SO₃H, NO₂, or CN, wherein:
R₃ and R₄, taken together with the carbon atoms to which they are attached, may form a phenyl group or a heterocyclic group; and R₇ and R₈, taken together with the carbon atoms to which they are attached, may form a phenyl group or a heterocyclic group;
L is a linking group, having a structure according to the following formula: 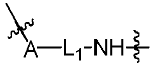
A is selected from the group consisting of S, N, and O, preferably, A is N or O;
L₁ is a carbon chain containing 8 to 20 carbon atoms, wherein the carbon atom(s) in the carbon chain may be replaced with oxygen atom(s) or nitrogen atom(s); the carbon chain can contain double bond(s); the hydrogen atom(s) in the carbon chain can be substituted with 1-5 R₁₃, wherein each R₁₃ is independently selected from benzyl, carboxyl, and C₁₋₃ alkyl, or two adjacent R₁₃ can form a ring, taken together with the atoms to which they are attached, and the carbon(s) in the carbon chain can be further replaced with carbonyl group(s); and
Y is a metal chelate.

2. The dual-modality contrast agent according to claim 1, wherein the X has the following structure: 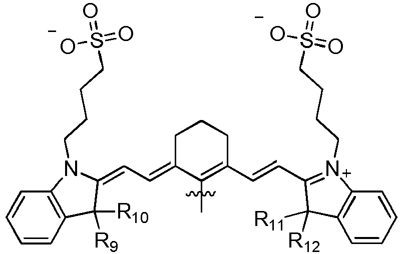 wherein R₉, R₁₀, R₁₁ and R₁₂ are defined as in claim 1.

3. The dual-modality contrast agent according to claim 1, wherein the X has the following structure:

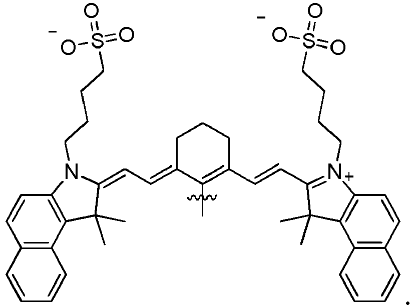

4. The dual-modality contrast agent according to claim 1, wherein the L has the following structure: 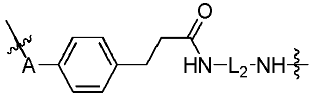
wherein the definition of A is the same as claim 1; and
L₂ has the same definition as L₁.

5. The dual-modality contrast agent according to claim 4, wherein the L₂ has the following formula: 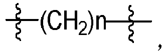 wherein n is 8-20.

6. The dual-modality contrast agent according to claim 1, wherein the metal chelate is complexed with Gd to form a Gd complex.

7. The dual-modality contrast agent according to claim 6, wherein the Gd complex has the following structures:

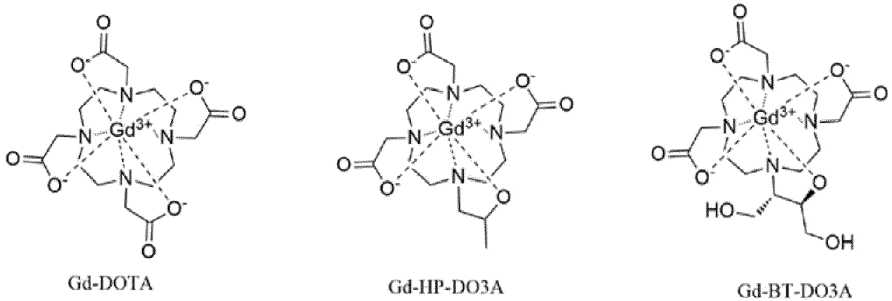

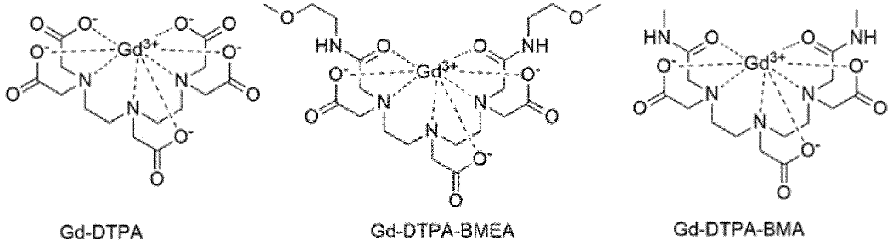

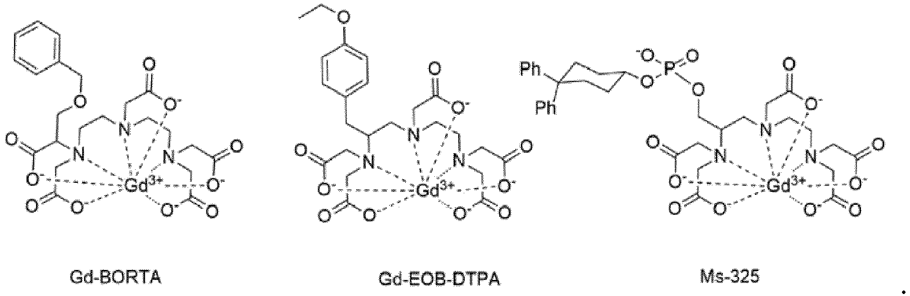

8. The dual-modality contrast agent according to claim 1, wherein the dual-modality contrast agent is selected from the following structures:

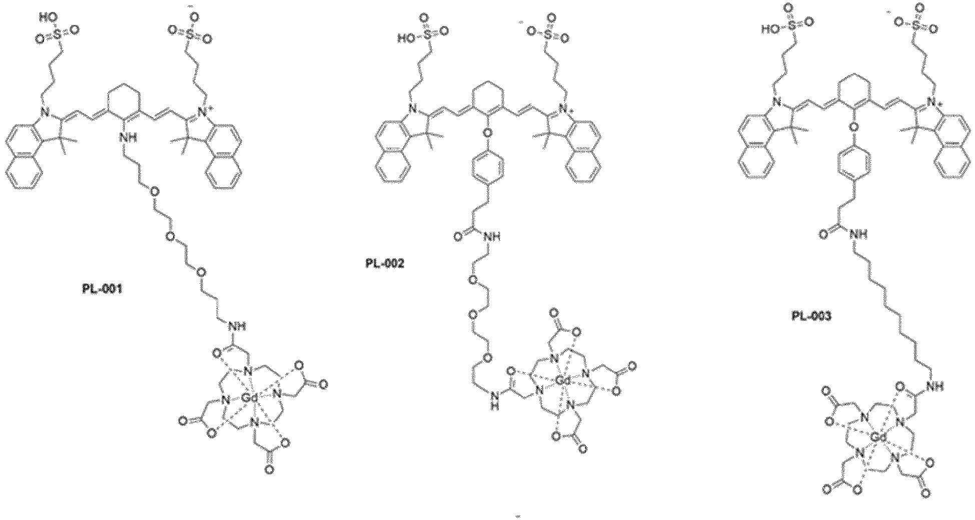

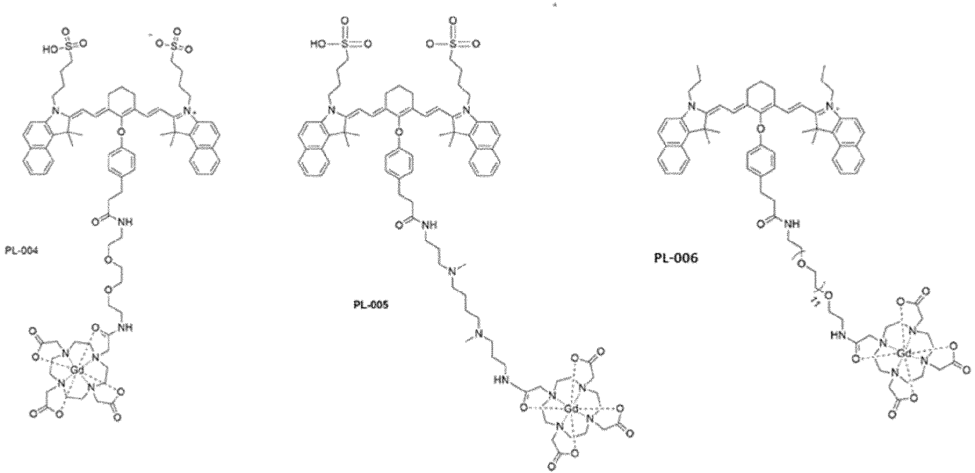

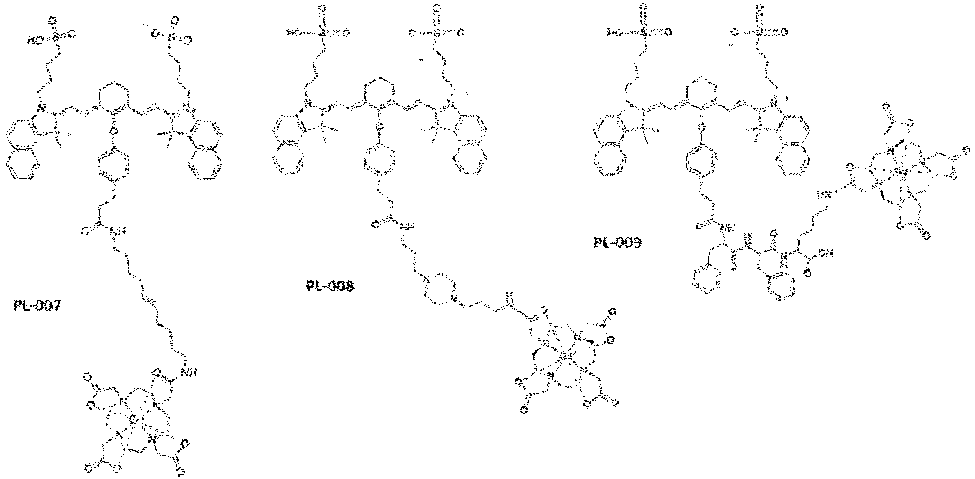

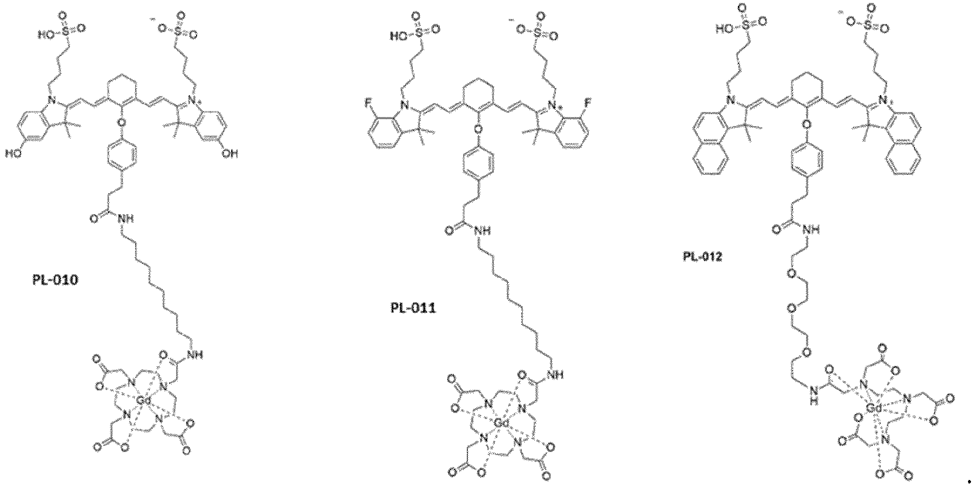

9. A method of preparing a fluorescence-magnetic resonance dual-modality contrast agent comprising the step of condensing a compound of formula I with a compound of formula II to obtain a fluorescence-magnetic resonance dual-modality contrast agent of formula III, 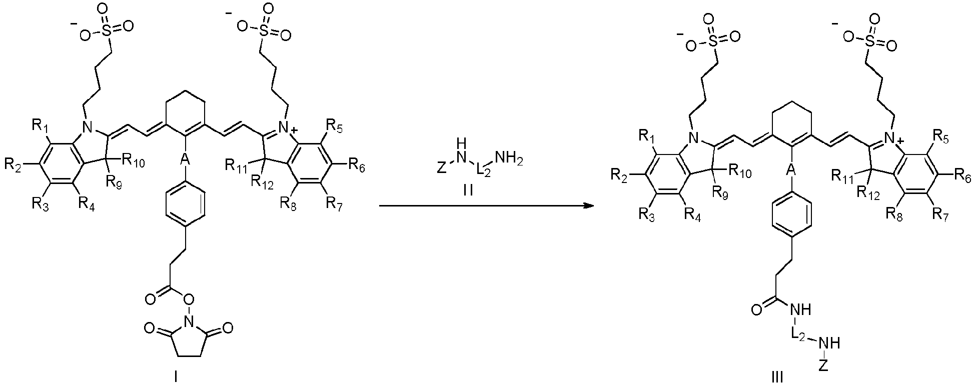
wherein the compound of formula II is obtained by complexing a compound of formula 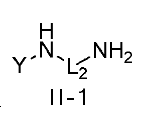 with gadolinium chloride hydrate,
wherein the definitions of R₁, R₂, R₃, R₄, R₅, R₆, R₇, R₈, R₉, R₁₀, R₁₁, R₁₂, Y, A, L, and Z are the same as defined in any one of claims 1-8;
alternatively, a compound of formula III-1 is complexed with gadolinium chloride hydrate to obtain a compound of formula III-2, 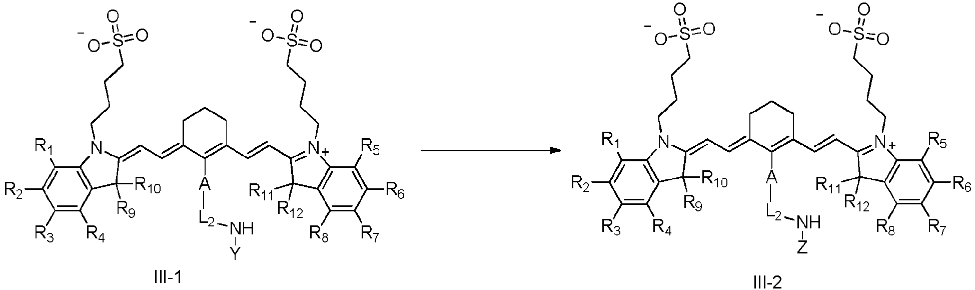
wherein a compound of formula I-2 is condensed with a compound of formula II-2 to obtain the compound of formula III-1, 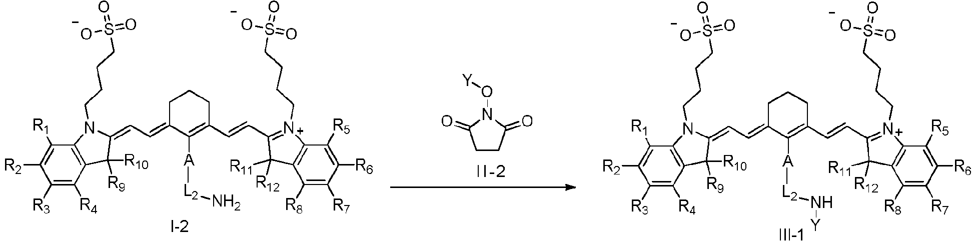
wherein R₁, R₂, R₃, R₄, R₅, R₆, R₇, R₈, R₉, R₁₀, R₁₁, R₁₂, A, L₂, and Y are the same as defined in any one of claims 1-8.

10. Use of a fluorescence-magnetic resonance dual-modality contrast agent in the preparation of a pharmaceutical composition intended for medical magnetic resonance-enhanced imaging, determination of liver function, assisting in preoperative planning, intraoperative fluorescence navigation, prediction of fluorescence distribution of viscera by magnetic resonance images, determination of liver function, determination of renal function, monitoring of the vivo circulation as well as labeling of cells, and markers for in vitro cellular analysis, wherein said fluorescence-magnetic resonance dual-modality contrast agent is the same as defined in any one of claims 1-8.
